# EUROPÄISCHE PATENTSCHRIFT

(11) **EP 1 836 911 B1**
(45) Veröffentlichungstag und Bekanntmachung des Hinweises auf die Patenterteilung: **02.06.2010**
(21) Anmeldenummer: 07002965.7
(22) Anmeldetag: 13.02.2007
(51) Int. Cl.: A41B 11/00, A43B 7/00

(54) **Kosmetische Fussbekleidung zur Hornhautentfernung**
Cosmetic sock for removing horny skin
Chaussette cosmétique destiné à l'élimination des callosités

(30) Priorität: 24.03.2006 DE 102006013535
(43) Veröffentlichungstag der Anmeldung: 26.09.2007
(73) Patentinhaber: Brändle, Thomas, 6850 Dornbirn (AT); Brändle, Hans-Jörg, 6850 Dornbirn (AT)
(72) Erfinder: Brändle, Thomas, 6890 Lustenau (AT); Brändle, Hans-Jörg, 6850 Dornbirn (DE)
(74) Vertreter: Riebling, Peter

(56) Entgegenhaltungen:
- JP-B1- 3 022 851
- US-A- 3 987 559
- US-A- 5 930 916

## Beschreibung

Gegenstand der Erfindung ist eine kosmetische Fußbekleidung zur Hornhautentfernung nach dem Oberbegriff des Patentanspruches 1.

Ein kosmetisches Verfahren zur Hornhaut- und Nagelhautentfernung mit nagelhauterweichenden Mitteln ist mit dem Gegenstand der DE 101 31 030 A1 bekannt geworden. Bei dieser bekannten Vorrichtung wird auf die zu entfernende Fußhautfläche eine Lotion aufgetragen, die nachfolgend mit einer Baumwollwatte und einer Kunststofffolie versiegelt wird. Nachteil dieses bekannten Verfahrens ist die schwierige Anwendung, die langwierige Behandlungszeit und der fragwürdige Behandlungserfolg.

Mit dem Gegenstand der DE 298 00 374 U1 ist ein universelles, beidseitig verwendbares Körperpflegeinstrument bekannt geworden, welches im wesentlichen aus einer Handhabungsvorrichtung mit einer vorderen Schleiffläche und einem daran ansetzenden Handgriff besteht. Mit einem solchen Instrument kann damit mit einer Schabewirkung an bestimmten Stellen an der Fußsohle die Hornhaut entfernt werden. Ein derartige Fussbekleidung ist aus der JP 03 022 851 B2 bekannt.

Der mit der vorliegenden Erfindung zu lösende Aufgabe kann somit darin gesehen werden: eine Socke mit einer höheren Elastizität und eine verbesserte Reibwirkung an bestimmten Stellen der Fußsohle zu erreichen.

Zur Lösung der gestellten Aufgabe ist die Erfindung durch die technische Lehre des Anspruches 1 gekennzeichnet.

Wesentliches Merkmal der Erfindung ist, dass mindestens eine der menschlichen Fußsohle zugewandte Fußbekleidung vorgesehen ist, auf der mindestens teilweise der Fußsohle zugewandte Schmirgelflächen angeordnet sind.

Mit der gegebenen technischen Lehre ergibt sich der wesentliche Vorteil, dass nun praktisch selbsttätig eine Entfernung von Hornhaut an der menschlichen Fußsohle und in den Nachbarbereichen stattfindet. Die Erfindung sieht eine Fußbekleidung vor, die stellenweise Schmirgelflächen aufweist, die gegen die menschliche Fußsohle gerichtet sind. Hierbei gibt es mehrere verschiedene Ausführungsformen, die alle vom Erfindungsgedanken der vorliegenden Erfindung umfasst sein sollen.

In einer ersten Ausgestaltung der Erfindung ist es vorgesehen, dass die Innenfläche einer Socke mit der erfindungsgemäßen Schmirgelbeschichtung versehen ist. Der Benutzer zieht sich eine derartige Socke an und zieht sich einen Schuh (Fußbekleidung) darüber. Mit zunehmender Laufleistung wirken die Schmirgelbereiche auf die menschliche Fußsohle, und Versuche haben gezeigt, dass gerade die Bereiche, die besonders druckbeansprucht sind, dazu neigen, Hornhaut in übermäßigem Maß zu entwickeln. Genau diese Bereich werden sozusagen selbsttätig durch die in diesen Bereichen angeordneten Schmirgelflächen an der Innenseite der Socke abgeschmirgelt, und es kommt damit zu einer selektiven Abtragung der Hornhaut nur an den Schichten, wo die größte Druckwirkung zwischen der Socke und der Gegenfläche entsteht.
Die Gegenfläche kann hierbei die Innensohle eines üblichen Schuhs sein, sie kann jedoch auch als Einlegesohle ausgebildet sein oder der Träger der Socke braucht nur auf einem Fußboden zu laufen, ohne dass er hierbei einen Schuh anhat.

Wichtig bei der Erfindung ist dem gemäß, dass es zu einer selbsttätigen Abtragung von Hornhautschichten an der Fußsohle kommt, je nachdem, wie lange der Träger diese kosmetische Socke benutzt und welche Druckwirkung er auf die Socke ausübt.

Ein schweres Körpergewicht mit einer dementsprechenden Socke wird also zu einer schnelleren Abtragung von Hornhaut führen, als vergleichsweise ein leichtes Körpergewicht, welches einen entsprechend geringeren Druck auf die Schmirgelschicht und die dagegen wirkende Gegenfläche ausübt.

Bei der Erfindung wird im übrigen bevorzugt, wenn nicht nur der Sohlenbereich der Socke mit entsprechenden Schmirgelflächen versehen ist, sondern - wie oben stehend ausgeführt - auch die Zehen- und die Fersenbereiche. Zu diesem Zweck kann es vorgesehen sein, dass die Socke in ihrem Innenbereich auch noch fersenseitig und zehenseitig beschichtet ist.

Ebenso kann die Schmirgelbeschichtung auch noch seitlich an der Socke entlanggeführt werden, um noch die Seitenbereich der Fußsohle mit einer entsprechenden abrasiven Wirkung zu versehen.

In einer zweiten Ausführungsform der vorliegenden Erfindung ist es vorgesehen, dass statt einer kosmetischen Socke eine Einlage mit den gleichen Schmirgelflächen versehen wird, wie sie anhand der vorstehend beschriebenen Socke beschrieben wurden.

Eine solche Einlage kann entweder in einen Schuh eingelegt werden oder sie kann auch als Sandalensohle unmittelbar als Sandale (Flip-Flop) ausgebildet sein.

Als Material für die Schmirgelpartikel können eine Vielzahl von Materialien verwendet werden. Insbesondere werden Korund-Partikel verwendet. Es können jedoch auch andere Reibkörper verwendet werden, die z. B. aus technischer Keramik gebildet sind und deren Zusammensetzung auch aus Glas, Aluminiumoxyd und Calcium besteht.

Neben den bekannten Korund-Kristallen oder Partikeln können somit auch andere grobkörnige und eine Raspel-Wirkung ausübende Partikel verwendet werden, insbesondere auch Glasig-, Kristaline-Alkali-, Erdalkali-, Alumosilicate.

Neben der erwünschten Massagewirkung kommt es zusätzlich zu einer Schmutzentfernung und einer Reinigung der Fußsohle und darüber hinaus - je nach Tragezeit - zu einer Abtragung von Hornhautflächen an den hochdruckbelasteten Stellen.

Die Körnung der Schmirgelpartikel kann in weiten Grenzen variiert werden. Bevorzugt werden 80ger-Körnungen, wie sie dem Körnungsmaß eines üblichen Schleifpapiers entsprechen.

Selbstverständlich können auch größere oder kleinere Körnungsmaße verwendet werden.

Eigene Versuche haben ergeben, dass die erfindungsgemäße Fußbekleidung oder Einlage mit der aufgebrachten Schmirgelbeschichtung waschbar ist, ohne dass sich die Schmirgelpartikel nennenswert aus der Beschichtung selbsttätig lösen.
Somit können eventuelle Verschmutzungen, wie zum Beispiel abgetragene Hornhautpartikel aus dieser Fußbekleidung oder Einlage entfernt werden.

Der Erfindungsgegenstand der vorliegenden Erfindung ergibt sich nicht nur aus dem Gegenstand der einzelnen Patentansprüche, sondern auch aus der Kombination der einzelnen Patentansprüche untereinander.

Alle in den Unterlagen, einschließlich der Zusammenfassung offenbarten Angaben und Merkmale, insbesondere die in den Zeichnungen dargestellte räumliche Ausbildung, werden als erfindungswesentlich beansprucht, soweit sie einzeln oder in Kombination gegenüber dem Stand der Technik neu sind.

Im Folgenden wird die Erfindung anhand von mehrere Ausführungswege darstellenden Zeichnungen näher erläutert. Hierbei gehen aus den Zeichnungen und ihrer Beschreibung weitere erfindungswesentliche Merkmale und Vorteile der Erfindung hervor.

Es zeigen:
- Figur 1:: Schnitt durch eine kosmetische Socke in einer ersten Ausführungsform der Erfindung
- Figur 2:: vergrößerter Teilschnitt in Richtung der Linie 2-2 in Figur 1
- Figur 3:: die Draufsicht auf den Sohlenbereich einer Socke nach Figur 1
- Figur 4:: Schnitt durch eine Einlage
- Figur 5:: stirnseitige Ansicht der Einlage nach Figur 4

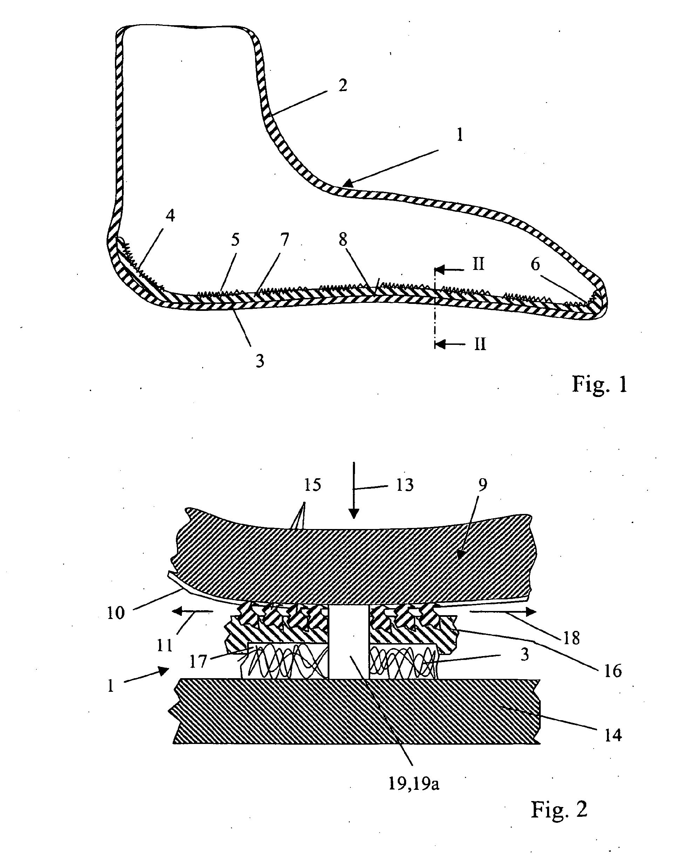

In Figur 1 ist schematisiert eine übliche Socke dargestellt. Eine solche Socke kann aus den verschiedenartigsten Materialien hergestellt werden. Beispielsweise werden hierfür Gestricke oder Gewirke verwendet, ebenso wie gefilzte oder gewalkte Textilmaterialien.

Es können ebenso auch Vlies-Stoffe oder Verbundstoffe aus gewirkten und/oder gestrickten Textilmaterialien verwendet werden.

Es können auch moderne feuchtigkeitsableitende und eine Membranwirkung aufweisende Textilmaterialien verwendet werden, die in der Regel aus technischen Geweben bestehen, die eine entsprechende Membranwirkung haben.

Es ist also im Rahmen der vorliegenden Erfindung gleichgültig, aus welchem Material die Socke besteht, denn es kommt im Rahmen der Erfindung lediglich darauf an, dass auf der Innenseite der Socke, und zwar an der fußzugewandeten Seite der Socke ein oder mehrere Schmirgelflächen 4, 5, 6 angeordnet sind, die unmittelbar auf die Fußsohle 9 (siehe Figur 2) wirken.

Im gezeigten Ausführungsbeispiel nach Figur 1 sind deshalb im Zehenbereich Schmirgelflächen 6, im mittigen Sohlenbereich Schmirgelflächen 5 und im Fersenbereich Schmirgelflächen 4 angeordnet, wobei die im Zehen- und im Fersenbereich angeordneten Schmirgelflächen 4, 6 noch hochgezogen sind, um auch die über die reine Sohle 3 hinausgehenden Flächen der menschlichen Fußsohle behandeln zu können.

Die Socke 1 hat im übrigen einen herkömmlichen Bund 2, so dass sich das gesamte Gewebe oder Gewirk um den Fuß herumschließt.

In Figur 2 sind weitere Einzelheiten der Anordnung dargestellt. Es ist gezeigt, dass die Schmirgelflächen 4, 5, 6 aus einzelnen Schmirgelpartikeln 15 bestehen, die in einer Klebeschicht 16 gebunden sind, wobei die Klebeschicht 16 aus einem ausgehärteten elastomeren Kunststoff besteht, z. B. einem Polyoretan, einem PVC und dergleichen Klebeschichten, die nach der Abbindung keine Klebewirkung mehr aufweisen.

Wichtig ist, dass die Schmirgelpartikel 15 möglichst fest in der Klebeschicht 16 und somit unverlierbar dort gehalten sind.

Die Klebeschicht 16 greift in einem Eindringbereich 17 in das Gewirk oder Strick der Sohle 3 der Socke 1 hinein und ist somit fest verbunden.

Dieser Verbindungsbereich (8) kann auch anders ausgebildet sein.

Die Figur 1 zeigt, dass zunächst auch eine Einlagesohle hergestellt werden kann, die über eine Trägerschicht 7 (Verbindungsschicht) mit der Innenseite der Socke 1 verbunden wird. Es kann sich hierbei um einen Klebeverbund oder um eine andere mechanische Befestigung, z. B. durch einzelne Fadenschlaufen und dergleichen handeln.

Es kann also dementsprechend auch eine Einlegesohle durch Kettelstiche mit der Innenseite der Socke 1 im Bereich deren Sohle 3 verbunden werden.

Der Verbindungsbereich 8 kann demzufolge als Klebeschicht oder als andere mechanische Verbindungsschicht ausgebildet sein.

Die Figur 2 zeigt nun die mechanische Wirkung der Schmirgelflächen 4-6. Wird aufgrund des Körpergewichtes in Pfeilrichtung 13 über die Fußsohle 9 ein Druck auf die Innenfläche der Socke 1 ausgeübt, dann wird dieser Druck zunächst auf die Schmirgelpartikel 15 übertragen, die aufgrund ihrer elastomeren Einbettung in die Klebeschicht 16 und aufgrund von Laufbewegungen nun laterale Bewegungen in den Pfeilrichtungen 11 und 12 ausführen. Damit kommt es zu einer abrasiven, abschälenden Wirkung im Bereich der Hornhaut 10 der Fußsohle 9. Damit wird die Hornhaut 10 gezielt an den Flächen abgetragen, wo der größte Druck herrscht, und erfahrungsgemäß ist dort auch der größte Hornhautabtrag gegeben.

Hierbei ist es gleichgültig, welche Auflagefläche 14 verwendet wird. Es kann die Socke 1 mit dem darin steckenden Fuß auf einem Fußboden aufliegen oder die Auflagefläche 14 kann durch die Innensohle eines Schuhs oder durch eine Einlegesohle gebildet sein.

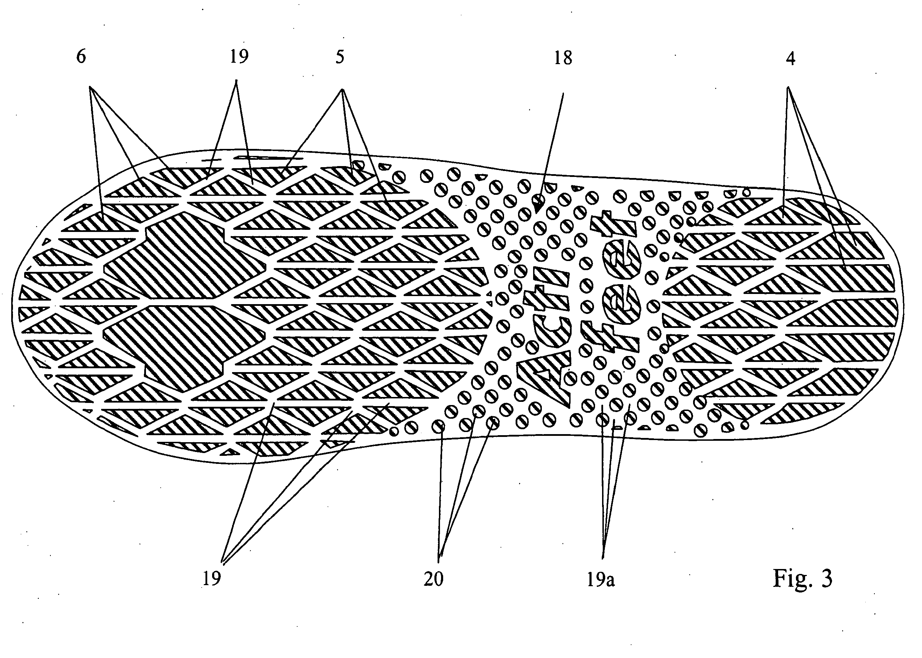

Die Figur 3 zeigt die Draufsicht auf die Innenfläche der Socke 1, wo erkennbar ist, dass sich Schmirgelflächen 4, 5, 6, 20 nur stellenweise aufgetragen sind. Es handelt sich um die dort gezeigten schwarzen Bereiche, die durch Unterbrechungsflächen 19, 19a voneinander getrennt sind.

Die Sockeninnenseite 18 ist also somit nur partiell mit den Schmirgelflächen 4-6, 20 versehen, um dazwischen Unterbrechungsflächen 19, 19a zu bilden, die aus dem textilen Material der Socke 1 selbst gebildet sind.

Dies heißt, die Schmirgelflächen zusammen mit den Klebeschichten 16 sind immer nur stückweise aufgetragen, so dass sich dazwischen unterbrochene Bereiche ergeben, die eine Verbesserung der Flexibilität der gesamten Socken-Innenseite 18 dienen. Durch die sich ergebenden Kanten in den Unterbrechungsflächen 19, 19a (siehe auch Figur 2) wird die abrasive Wirkung der Partikel verbessert und im übrigen auch ein verbesserter Feuchte- und Schmutzdurchgang durch die Unterbrechungsflächen 19, 19a gewährleistet.

Es kommt damit zur Ablagerung von abgeschliffenen und abgetragenen Hornhautbereichen in den durchgehenden Unterbrechungsflächen 19, 19a, die bis zum Grund der Sohle 3 der Socke 1 hindurchgehen.

Diese Unterbrechungsflächen 19, 19a können sich somit elastisch aufweiten oder verengen und bilden so eine ausgezeichnete Elastizität für die gesamte Socke-Innenseite 18 aus.

Die Figur 3 zeigt im übrigen, dass die Unterbrechungsflächen 19, 19a durchaus unterschiedlich ausgebildet sein können und dass demzufolge auch die zugeordneten Schmirgelflächen 4, 5, 6, 20 unterschiedlich dimensioniert sein können.

Im vorderen Bereich (Ballenbereich) der Fußsohle sind somit die größer angelegten Schmirgelflächen 4-6 ausgebildet, während im Fußgewölbebereich - wo nur eine geringe Abtragungswirkung oder gar keine Abtragungswirkung erwünscht ist - sind hingegen punktförmige Schmirgelflächen 20 angeordnet, die zwischen sich relativ große Unterbrechungsflächen 19a bilden.

Im Fersenbereich, wo wiederum große Hornhautbeläge vorhanden sind, sind wieder großflächige Schmirgelflächen 4 vorgesehen.

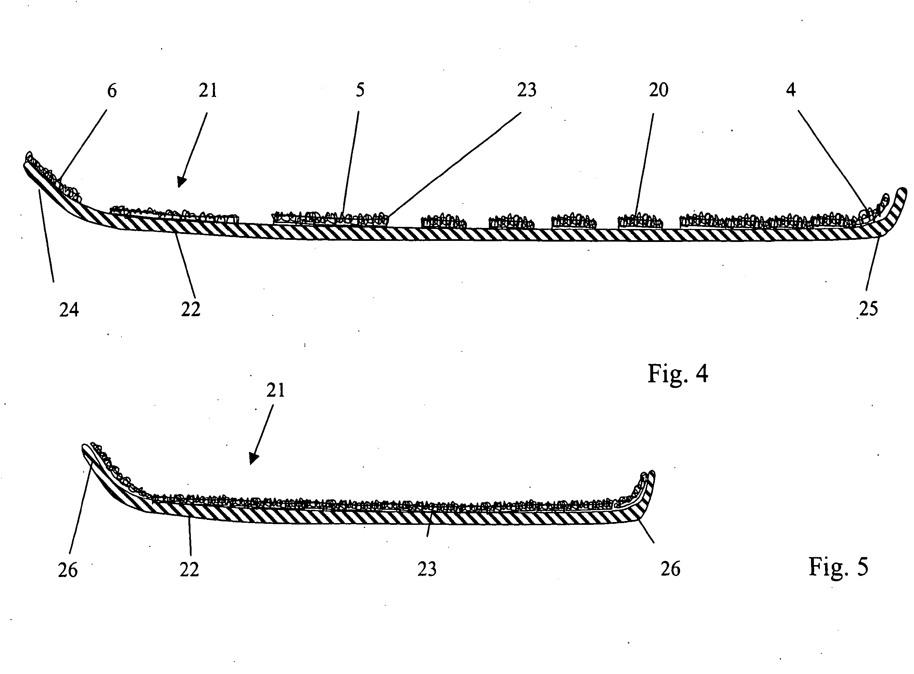

In Figur 4 und 5 ist die Ausbildung einer Socke 1 nur als Einlage 21 dargestellt. Eine solche Einlage kann - entsprechend dem allgemeinen Beschreibungsteil - entweder als Sandale ausgebildet sein oder als Einlage in einen üblichen Schuh.

Sie kann lose eingelegt werden oder sie kann nachträglich fest in die Innenseite des Schuhs eingeklebt werden.

Die Einlage besteht wiederum aus einer üblichen, elastomeren Trägerschicht 22, die aus Kunststoff oder aus Leder oder aus einem anderen geeigneten Material bestehen kann.

Auf die Oberfläche der Trägerschicht 22 ist eine Klebeschicht 23 angeordnet, auf der die vorher genannten Schmirgelflächen 4-6, 20 angeordnet sind.

Auch hier ist wichtig, dass die Einlage einen hochgezogenen Zehenbereich 24 und ebenfalls einen hochgezogenen, hinteren Fersenbereich 25 aufweist, um auch in diesen Bereichen eine entsprechende Abtragungswirkung auf die menschliche Fußsohle zu erreichen.

Die Figur 5 zeigt zusätzlich, dass auch die Seitenbereiche 26 der Einlage 21 hochgezogen sind, um auch die seitlichen Fußsohlenbereiche einer Abtragungswirkung zu unterziehen.

Allen beiden Ausführungsbeispielen ist gemeinsam, dass es zu einer selbsttätigen Abtragung von Hornhaut nur an den hochbelasteten Stellen der menschlichen Fußsohle kommt und dass damit ein besonders einfacher kosmetischer, schnell wirkender Hornhaut abtragender Effekt erzielt wird.

Je nach Hornhautstärke und nach Körpergewicht reichen einige Tage aus, um auch starke Hornhautbeläge abzutragen. Selbstverständlich umfasst die vorliegende Erfindung auch Möglichkeiten, die Socke und insbesondere die Schmirgelflächen und deren dazwischen liegende Bereiche zu imprägnieren, mit Duftstoffen zu versehen und mit anderen Mitteln noch zusätzlich zu beschichten.

Ebenso kann es vorgesehen sein, die Schmirgelschichten selbst mit einer antibakteriellen Silberschicht zu versehen, um noch eine verbesserte Heilungswirkung - insbesondre bei pathologischen Erscheinungen, wie z. B. Fußpilz, Stechwarzen, entzündete Schwielen und dergleichen mehr zu erreichen.

### Zeichnungslegende

- 1.: Socke
- 2.: Bund
- 3.: Sohle
- 4.: Schmirgelfläche (Ferse)
- 5.: Schmirgelfläche (Sohle)
- 6.: Schmirgelfläche (Zehe)
- 7.: Trägerschicht
- 8.: Verbindungsbereich
- 9.: Fußsohle
- 10.: Hornhaut
- 11.: Pfeilrichtung
- 12.: Pfeilrichtung
- 13.: Pfeilrichtung
- 14.: Auflagefläche
- 15.: Schmirgelpartikel
- 16.: Klebeschicht
- 17.: Eindringbereich
- 18.: Socken-Innenseite
- 19.: Unterbrechungsfläche 19a
- 20.: Schmirgelfläche (Gewölbe)
- 21.: Einlage
- 22.: Trägerschicht
- 23.: Klebeschicht
- 24.: Zehenbereich
- 25.: Fersenbereich
- 26.: Seitenbereich

## Patentansprüche

1. Kosmetische Fußbekleidung bestehend aus einer textilen Socke, auf deren dem menschlichen Fuß zugewandte Innenseite mittels Klebeschichten punktförmige Vorsprünge zur Hornhautentferhung aufgebracht sind, die als Schmirgelflächen (4, 5, 6, 20) im Zehen-, Fersen- und Seitenbereich (24, 25, 26) des zu behandelnden Fußes ausgebildet sind, wobei die Schmirgelflächen (4, 5, 6, 20) mit Klebeschichten einheitlich über die gesamte Oberfläche mit dazwischen liegenden Unterbrechungsflächen (19, 19a) auf dem textilen Material der Socke (1) angeordnet sind, **dadurch gekennzeichnet, dass** im vorderen Bereich (Ballenbereich) der Fußsohle die größer angelegten Schmirgelflächen (4-6) ausgebildet sind, während im Fußgewölbebereich - wo nur eine geringe Abtragungswirkung oder gar keine Abtragungswirkung erwünscht ist - hingegen punktförmige Schmirgelflächen (20) angeordnet sind, die zwischen sich relativ große Unterbrechungsflächen (19, 19a) bilden.

2. Kosmetische Fußbekleidung nach Anspruch 1, **dadurch gekennzeichnet, dass** eine mechanische Wirkung der Schmirgelflächen (4, 5, 6, 20) aufgrund des Körpergewichtes über die Fußsohle (9) einen Druck auf die Innenfläche der Socke (1) erzeugt, welcher sich auf die Schmirgelpartikel (15) überträgt, die aufgrund ihrer elastomeren Einbettung in eine Klebeschicht (16) und aufgrund von Laufbewegungen laterale Bewegungen in den Pfeilrichtungen (11) und (12) ausführen, wodurch eine abrasive, abschälende Wirkung im Bereich der Hornhaut (10) der Fußsohle (9) ausgebildet ist.

3. Kosmetische Fußbekleidung nach einem der vorhergehenden Ansprüche 1 bis 2, **dadurch gekennzeichnet, dass** die zur Bildung der Schmirgelflächen (4, 5, 6) zu verwendende Schmirgelpartikel (15) als Korund-Partikel, technische Keramik, Glas, Aluminiumoxid und Calcium, Silikat-Verbindungen oder dergleichen ausgebildet sind, welche mit einer Klebeschicht (16) unverlierbar gebunden sind und nach dessen Abbindung eine Klebewirkung ausschließen.

4. Kosmetische Fußbekleidung nach Anspruch 3, **dadurch gekennzeichnet, dass** die Körnung der Schmirgelpartikel eine 80er-Körnung ist, wie sie dem Körnungsmaß eines üblichen Schleifpapiers entspricht.

5. Kosmetische Fußbekleidung nach einem der vorhergehenden Ansprüche 1 bis 4, **dadurch gekennzeichnet, dass** die Klebeschicht (16) in einem Eindringbereich (17) in das Gewirk oder Strick der Sohle (3) der Socke (1) eingreift.

6. Kosmetische Fußbekleidung nach einem der vorhergehenden Ansprüche 1 bis 5, **dadurch gekennzeichnet, dass** die partiellen Schmirgelflächen (4, 5, 6) zusätzlich zur abrasiven Abtragung der Hornhaut (10) eine Massage- und Reinigungsfunktion aufweisen, wobei die Unterbrechungsflächen (19, 19a) einen verbesserten Feuchte- und Schmutzdurchgang ausbilden.

7. Kosmetische Fußbekleidung nach einem der vorhergehenden Ansprüche 1 bis 6, **dadurch gekennzeichnet, dass** die Socke (1) als Gestrick und/oder Gewirk, als gefilztes oder gewalktes Textilmaterial, als Vlies- oder Verbundstoff aus gewirkten und/oder gestrickten Textilmaterial oder dergleichen, welche aus feuchtigkeitsableitenden und/oder Membranwirkung aufweisende Textilmaterialien gebildet sind.

8. Kosmetische Fußbekleidung nach einem der vorhergehenden Ansprüche 1 bis 7, **dadurch gekennzeichnet, dass** die Schmirgelflächen (4, 5, 6, 20) mit einer antibakteriellen Silberschicht überziehbar sind.

## Claims

1. Cosmetic footwear, consisting of a textile sock, on the inside of which facing the human foot, punctiform projections are applied by means of adhesive layers to remove horny skin and are arranged as emery areas (4, 5, 6, 20) in the toe, heel and side region (24, 25, 26) of the foot to be treated, the emery areas (4, 5, 6, 20) being uniformly arranged using adhesive layers over the entire surface with interruption areas (19, 19a) located in between on the textile material of the sock (1), **characterised in that** the larger emery areas (4-6) are configured in the front region (ball region) of the sole of the foot, while arranged in the arch region of the foot - where only a small removal action or no removal action at all is desired - are punctiform emery areas (20), on the other hand, which form relatively large interruption areas (19, 19a) between them.

2. Cosmetic footwear according to claim 1, **characterised in that** a mechanical action of the emery areas (4, 5, 6, 20), because of the body weight over the sole (9) of the foot, produces a pressure on the inner area of the sock (1), which transfers to the emery particles (15), which because of their elastomeric embedding in an adhesive layer (16) and because of walking movements, carry out lateral movements in the arrow directions (11) and (12), so an abrasive, exfoliating effect is formed in the region of the horny skin (10) of the sole (9) of the foot.

3. Cosmetic footwear according to any one of claims 1 to 2, **characterised in that** the emery particles (15) to be used to form the emery areas (4, 5, 6), are configured as corundum particles, technical ceramic, glass, aluminium oxide and calcium, silicate compounds or the like, which are non-detachably bound to an adhesive layer (16) and after the setting thereof, rule out an adhesive effect.

4. Cosmetic footwear according to claim 3, **characterised in that** the grain size of the emery particles is an 80 grain size, as corresponds to the grain size measure of conventional sandpaper.

5. Cosmetic footwear according to any one of claims 1 to 4, **characterised in that** the adhesive layer (16) engages in a penetration region (17) in the knitted or interlaced fabric of the sole (3) of the sock (1).

6. Cosmetic footwear according to any one of claims 1 to 5, **characterised in that** the partial emery areas (4, 5, 6), in addition to the abrasive removal of horny skin (10), have a massage and cleaning function, the interruption areas (19, 19a) forming improved passage for moisture and dirt.

7. Cosmetic footwear according to any one of claims 1 to 6, **characterised in that** the sock (1) is formed as an interlaced and/or knitted fabric, as a felted and/or milled textile material, as a nonwoven or composite material made of knitted and/or interlaced textile material or the like, which are formed from textile materials which have a moisture-removing and/or a membrane effect.

8. Cosmetic footwear according to any one of claims 1 to 7, **characterised in that** the emery areas (4, 5, 6, 20) can be coated with an antibacterial silver layer.

## Revendications

1. Pièce d'habillement cosmétique pour le pied composée d'une chaussette textile dont le côté intérieur tourné vers le pied de la personne est recouvert, à l'aide de couches de colle, de saillies en forme de points qui sont destinées à l'élimination des callosités et qui sont conçues comme des surfaces abrasives (4, 5, 6, 20) dans la zone des orteils, du talon et des côtés (24, 25, 26) du pied à traiter, les surfaces abrasives (4, 5, 6, 20) avec les couches de colle étant disposées sur le matériau textile de la chaussette (1) de manière uniforme sur toute la surface, avec entre elles des surfaces d'interruption (19, 19a), **caractérisée en ce qu'**il est prévu dans la zone avant (zone des coussinets) de la plante du pied les surfaces abrasives (4-6) les plus grandes, tandis que dans la zone de la voûte plantaire - où on ne souhaite qu'une faible action d'élimination, voire pas d'action d'élimination du tout - il est prévu au contraire des surfaces abrasives sous forme de points (20) qui forment entre elles des surfaces d'interruption (19, 19a) relativement grandes.

2. Pièce d'habillement cosmétique pour le pied selon la revendication 1, **caractérisée en ce qu'**une action mécanique des surfaces abrasives (4, 5, 6, 20) due au poids du corps produit par l'intermédiaire de la plante du pied (9) une pression sur la surface intérieure de la chaussette (1) qui se transmet aux particules abrasives (15), lesquelles, en raison de leur insertion élastomère dans une couche de colle (16) et en raison des mouvements de la marche, décrivent des mouvements latéraux dans le sens des flèches (11) et (12), produisant ainsi une action abrasive de desquamation dans la zone des callosités (10) de la plante du pied (9).

3. Pièce d'habillement cosmétique pour le pied selon l'une des revendications précédentes 1 à 2, **caractérisée en ce que** les particules abrasives (15) à utiliser pour former les surfaces abrasives (4, 5, 6) sont conçues comme des particules de corindon, de la céramique technique, du verre, de l'oxyde d'aluminium et du calcium, des composés de silicate ou autres, qui sont reliés de manière imperdable à une couche de colle (16) et excluent, après le détachement de celle-ci, une action de collage.

4. Pièce d'habillement cosmétique pour le pied selon la revendication 3, **caractérisée en ce que** la granulométrie des particules abrasives est une granulométrie 80 telle qu'elle correspond à la granulation d'un papier émeri classique.

5. Pièce d'habillement cosmétique pour le pied selon l'une des revendications précédentes 1 à 4, **caractérisée en ce que** la couche de colle (16) pénètre dans le tissu à mailles ou le tissu tricoté de la semelle (3) de la chaussette (1) dans une zone de pénétration (17).

6. Pièce d'habillement cosmétique pour le pied selon l'une des revendications précédentes 1 à 5, **caractérisée en ce que** les surfaces abrasives partielles (4, 5, 6) présentent, en plus de l'élimination par abrasion des callosités (10), une fonction de massage et de nettoyage, les surfaces d'interruption (19, 19a) formant un passage amélioré pour l'humidité et la saleté.

7. Pièce d'habillement cosmétique pour le pied selon l'une des revendications précédentes 1 à 6, **caractérisée en ce que** la chaussette (1) est conçue comme un tissu tricoté et/ou un tissu à mailles, un matériau textile feutré ou foulé, un mat ou un matériau composite composé d'un matériau textile à mailles et/ou tricoté ou autre, qui sont formés à partir de matériaux textiles évacuant l'humidité et/ou présentant une action de membrane.

8. Pièce d'habillement cosmétique pour le pied selon l'une des revendications précédentes 1 à 7, **caractérisée en ce que** les surfaces abrasives (4, 5, 6, 20) sont aptes à être recouvertes d'une couche d'argent antibactérienne.
